# EUROPEAN PATENT APPLICATION

(11) **EP 1 983 054 A1**
(43) Date of publication of application: **22.10.2008**
(21) Application number: 08007445.3
(22) Date of filing: 16.04.2008
(51) Int. Cl.: C12N 15/60, C12N 9/88, C12N 15/63, C12N 1/21, A61K 38/51, A61K 31/713, G01N 33/50

(54) **Carbonic anhydrase and methods using the same**

(30) Priority: 20.04.2007 EP 07008083
(71) Applicant: Georg-August-Universität Göttingen, 37073 Göttingen (DE)
(72) Inventor: Wörhelde, Gert, Jun. Prof. Dr., 37086 Göttingen (DE); Jackson, Daniel, Dr., Brisbane (AU); Macis, Luciana, 37120 Bovenden (DE)
(74) Representative: Kröncke, Rolf

(57) **Abstract**

The present invention relates to methods for degrading carbon dioxide as well as to methods for synthesising carbon dioxide using a polypeptide having carbonic anhydrase activity. In particular, the present invention pertains to methods using carbonic anhydrase derived from *Astrosclera willeyana* or polypeptides similar thereto having carbonic anhydrase activity and being able to degrade carbon dioxide. Further, the present invention relates to methods to capture CO₂ and methods for identifying inhibitors or activators of said polypeptide having carbonic anhydrase activity. Finally, the present invention pertains to the polypeptide itself as well as to nucleic acid molecules encoding the same and vectors and host cells containing said nucleic acid molecules.

## Description

The present invention relates to methods for degrading carbon dioxide as well as to methods for synthesising carbon dioxide using a polypeptide having carbonic anhydrase activity. In particular, the present invention pertains to methods using carbonic anhydrase derived from *Astrosclera willeyana* or polypeptides similar thereto having carbonic anhydrase activity and being able to degrade carbon dioxide. Further, the present invention relates to methods to capture CO₂ and methods for identifying inhibitors or activators of said polypeptide having carbonic anhydrase activity. Finally, the present invention pertains to the polypeptide itself as well as to nucleic acid molecules encoding the same and vectors and host cells containing said nucleic acid molecules.

### Background art

Carbonic anhydrase (EC4.2.1.1.) is a globular zinc metalloenzyme. In humans, said enzyme has a molecular weight of about 30 kDa. Firstly described in 1933, nowadays various isoforms have been identified in plant and animal tissues where it is believed to facilitate the transport of carbon dioxide. For example, red blood cells contain isoenzymes I and II, which are the most active. Carbonic anhydrase II has the highest turnover number of any known enzymes. Physiologically, carbonic anhydrase facilitates the removal of carbon dioxide from the environment. The general enzyme reaction is shown below:

CO₂+ H₂O ↔ H⁺ HCO₃⁻

A milestone in the evolution of complex multicellular life was the evolution of the capacity to catalyse the reversible hydration of CO₂. This single reaction allows living cells to process metabolic wastes, regulate pH, fix carbon and transport ions across cellular and organelle membranes. The metalloenzyme carbonic anhydrase (CA) is pivotal to this process as it catalyses this reaction approximately one million fold and is found in organisms ranging from cyanobacteria to mammals.

For example, US 2006/0257990 describes carbonic anhydrase having increased stability under high temperature conditions. In addition, carbonic anhydrase derived from common oyster is described in JP 2006-262726.

Further, it is known that carbonic anhydrase also has an activity to degrade amorphous or crystalline silicon dioxides or silicones and other silicon or metal compounds, and mixed polymers of these compounds as well as synthesizing said compounds dependent on the reactants.

Besides metabolic activity of reducing or stabilising the pH in cellular systems, the carbonic anhydrase is described as a target for treating various diseases, e.g. cancer or obesity. Further, the use of inhibitors of carbonic anhydrase as antiglaucoma drugs has been discussed. In addition, the use of certain carbonic anhydrase isoenzymes, e.g. CA IX in humans as marker of tumour hypoxia and its predictive and prognostic potential has been demonstrated in numerous clinical studies.

Further, the increasing presence of carbonic dioxide in the environment contributes to the greenhouse effect. Various methods for the recovery or the conversion of CO₂ into carbonate and hydrogen and have been developed recently. For example US-patent 6,524,843 described the use of carbonic anhydrase in a system of gas extraction comprising carbon dioxide. Carbonic anhydrase facilitates the CO₂ transfer from gaseous to liquid phase following well known law governing gas mass transfer. E.g. US Patent Application No. 2004-0219090 disclosed a process to remove CO₂ from a gaseous steam an turn it into a stable solid form. Said process comprises passing CO₂ in rich gas through a gas diffusion membrate to transfer the CO₂ to a fluid medium. The CO₂ rich fluid is then passed through a matrix containing carbonic anhydrase to accelerate the conversion of CO₂ to carbonic acid. In the final step, a mineral ion is added to the reaction so that a precipitate of carbonate salts is formed. However, various drawbacks are associated with said methods.

Furthermore, carbonic anhydrase and its analogues proved able to function efficiently in dealing with the composition of the capturing solution and with pollutants (like SOx, NOx, lead, mercury or arsenic ions) that can be found in exhaust gases.

To conclude, carbonic anhydrase seems to be a promising tool to capture CO₂ in fluids. Further, carbonic anhydrase represents a target for treating various diseases and disorders.

However, the carbonic anhydreases described so far have various drawbacks regarding availability, stability and efficiency.

Coralline sponges (e.g. Stromatoporoids), chaetetids, sphinctozons are members of the earliest branching metazoan taxon (Porifera) to secret a CaCO₃ skeletons, and were major contributors to reef building processes in the late Palaeozoic and Mesozoic eras. A few of theses taxa survived today. The living fossil *Astrosclera willeyana* (a coralline marine and demosponge with a stromatoporid-like bodyplan) constructs a secondary basal skeleton of solid aragonite (CaCO₃) in addition to its primary spicules and is present in the fossil record from the late Triassic. Thus, in contrast to most soft-bodies sponges, coralline sponges, also called sclerosponges, secret the secondary rigid calcium carbonate skeleton.

Thus, these coralline-sponges are not only able to develop silicon dioxide skeletons, but also CaCO₃-sketetons.

The object of the present invention was to provide new carbonic anhydrase enzymes able to degrade carbon dioxide in vitro and in vivo. Said carbonic anhydrase enzymes are also able to degrade amorphous or crystalline silicon dioxide or silicones and other silicon (IV)- or metal (IV)-compounds as well as mixed polymers of these compounds.

### Summary of the present invention:

The present invention provides novel carbonic anhydrase enzymes. In particular, polypeptides are provided having carbonic anhydrase activity, being able to degrade carbon dioxide and/or amorphous or crystalline silicon dioxide having a sequence similarity of at least 50% to the sequence shown in SEQ ID Nos. 2, 4 and 5, respectively.

In a preferred embodiment, the sequence similarity is at least 70%, e.g. 80%, 90%, 92%, 95%, 97%, 98% and particular preferable 99%.

In a further embodiment, the present invention relates to nucleic acid molecules encoding the polypeptides according to the present invention.

Furthermore, the present invention pertains to vectors containing said nucleic acid molecules as well as to host cells containing said vectors.

In addition, the present invention relates to methods for in vitro or in vivo degradation of carbon dioxide and/or amorphous or crystalline silicon dioxide or silicones and other silicon (IV)- or metal (IV)-compounds as well as of mixed polymers of these compounds wherein a polypeptide or a metal complex of a polypeptide is used for the degradation wherein the polypeptide comprises a carbonic anhydrase activity and exhibits a sequence similarity of at least 50% to the sequence shown in SEQ ID No. 2, 4 and 5, respectively, and said peptide is able to degrade carbon dioxide and/or amorphous or crystalline silicon dioxide.

Further, a method is described for the synthesis of carbon dioxide and/or amorphous or crystalline silicone dioxide, silicones and other silicon (IV)- or metal (IV)-compounds as well as of mixed polymers of these compounds, wherein a polypeptide or a metal complex of a polypeptide is used for the synthesis, characterised in that said polypeptide has a carbonic anhydrase activity and has at least 50% sequence similarity to the sequence shown in SEQ ID Nos. 2, 4 and 5, respectively, and said polypeptide is able to degrade carbon dioxide and/or amorphous or crystalline silicon dioxide.

The polypeptide having carbonic anhydrase activity is useful in methods for identifying inhibitors or activators of said polypeptide. Further, pharmaceutical preparations containing said polypeptides or said inhibitors or activators identified using said polypeptides are envisages.

### Brief description of the figures

### Fig. 1

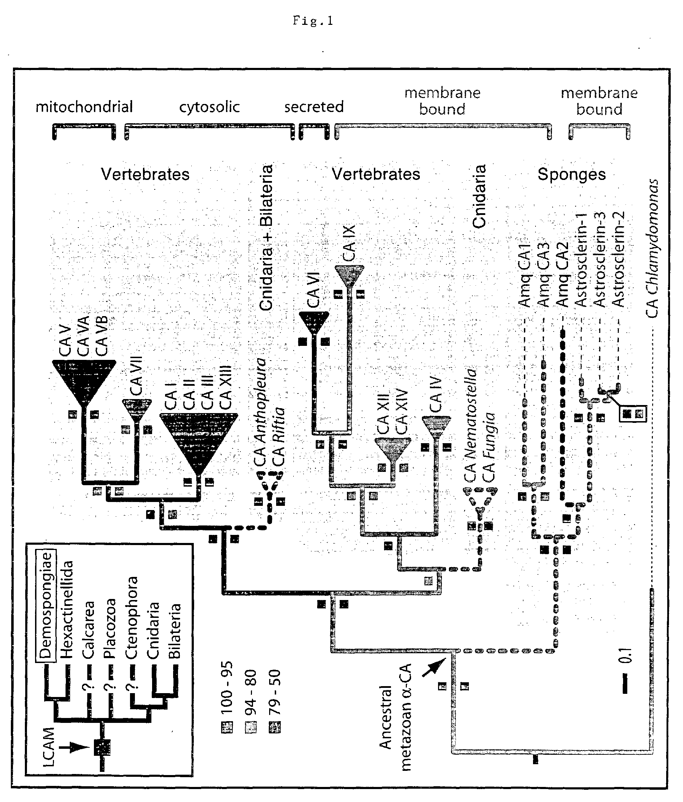

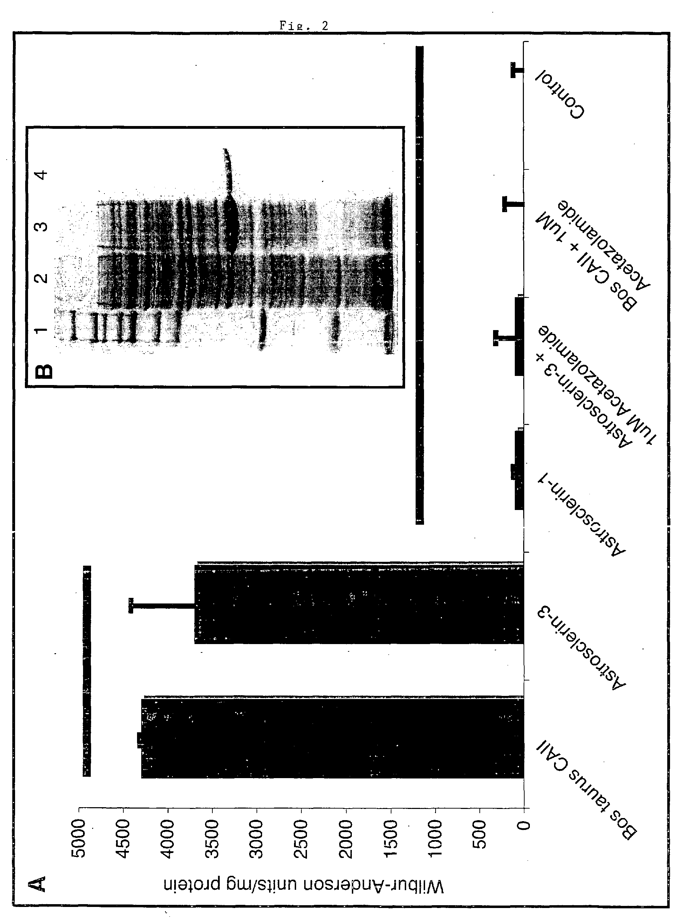

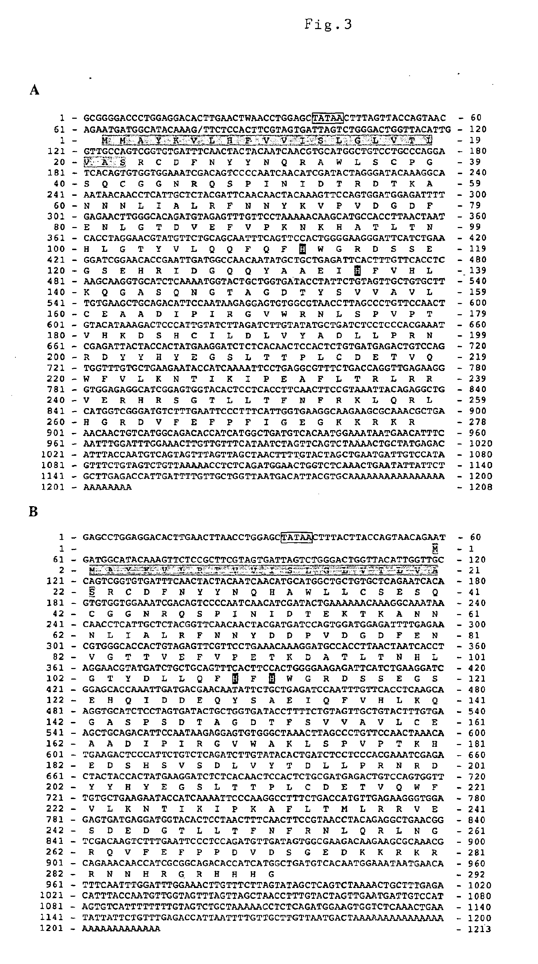

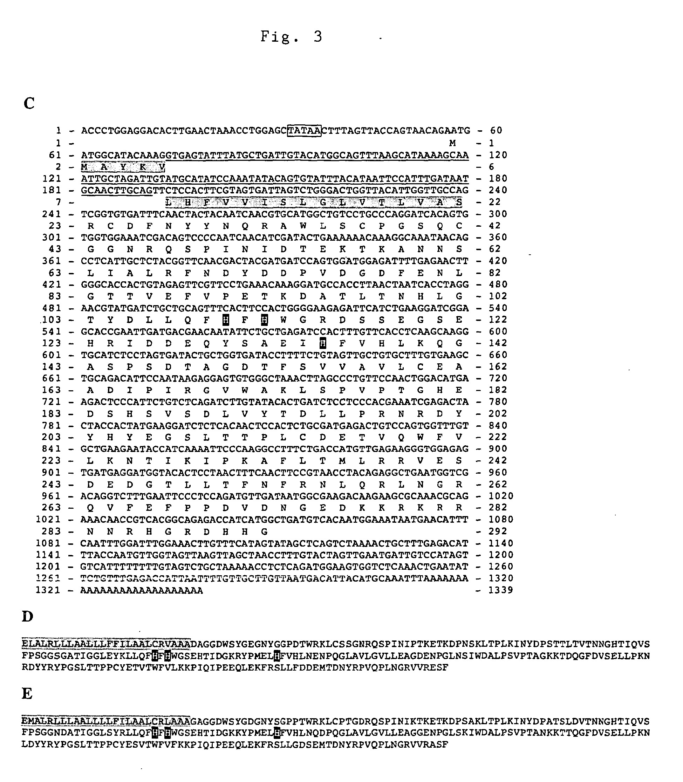

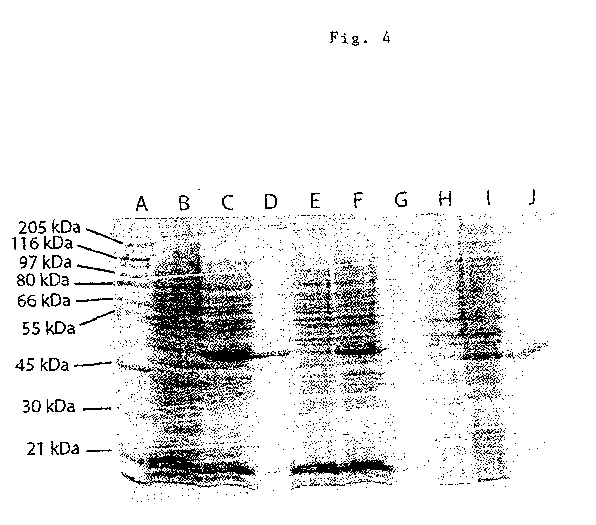

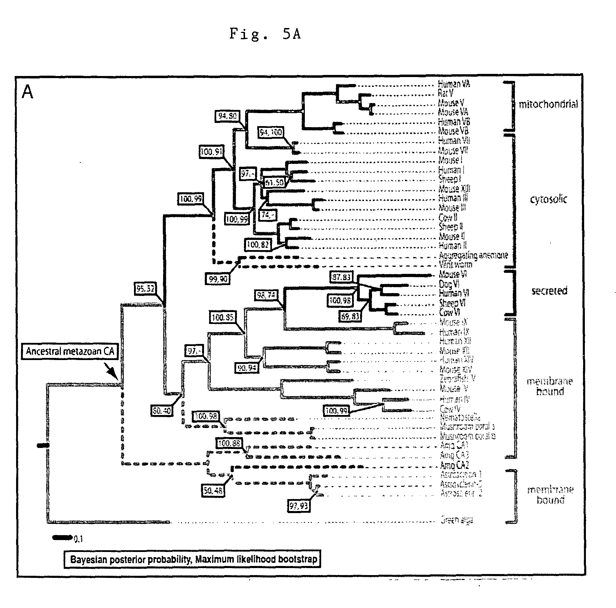

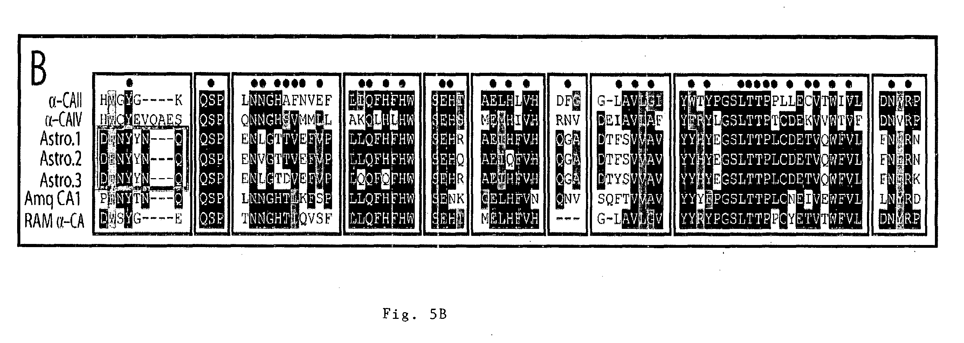

Phylogenetic analysis of the α carbonic anhydrase (α-CAs). Inset is a simplified phylogeny of the Metazoa. A. willeyana is a member of the early branching Demospongiae. Bayesian and Maximum Likelihood methods were used to reconstruct the evolutionary history of the α-CAs, with a non-metazoan α-CA as an outgroup. The tree topology shown here is derived from a Bayesian analysis with posterior probalities (above branch lines) and maximum likelihood bootstrap values (below branch lines) indicated as coloured squares. The tips of the tree have been collapsed for clarity of presentation (see Fig. 5 for a fully resolved tree). Experimentally validated sub-cellular localizations are indicated by solid colored lines. Predicted or inferred localizations are indicated by dashed lines.

### Fig. 2

Wilbur-Anderson activity assay and overexpression of Astrosclerin-3. (A) Activity of Astrosclerin-1 and -3 and bovine CAII in the presence and absence of the inhibitor Acetazolamide expressed in Wilbur-Anderson units/mg protein. Horizontal bars that group columns indicate no significant difference (ANOVA α=0,05, N=3) (B) SDS-PAGE of un-induced (lane 2) and induced (lane 3) Origami B (DE3) cells harbouring the pET16b plasmid with Astrosclerin-3 insert. Lane 4 shows affinity purified Astrosclerin-3 protein and lane 1 a protein standard.

### Fig. 3.

Full length cDNA and derived protein sequences of the *Astrosclerins* and reconstructed ancestral α-CA. Introns are underlined and signal peptides are highlighted grey, histidine residues involved in coordinating the catalytic zinc ion are highlighted in black and putative TATA boxes are boxed. (A) Full length cDNA encoding *Astrosclerin-1.* (B) Full length cDNA encoding *Astrosclerin-2.* (C) Full length cDNA encoding *Astrosclerin-3.* (D) Reconstruction of the ancestral metazoan α-CA by maximum likelihood (marginal reconstruction). (E) Reconstruction of the ancestral metazoan α-CA by maximum likelihood (joint reconstruction).

### Fig. 4

SDS-PAGE profiles of Astrosclerin-3, -2 and -1 over-expressed in Origami B (DE3) cells. (A) Molecular weight marker. (B) Un-induced cells with Astrosclerin-3 pET16b plasmid. (C) Induced cells with Astrosclerin-3 pET16b plasmid. (D) Affinity purified Astrosclerin-3. (E) Un-induced cells with Astrosclerin-2 pET16b plasmid. (F) Induced cells with Astrosclerin-2 pET16b plasmid. (G) Affinity purified Astrosclerin-2. (H) Un-induced cells with Astrosclerin-1 pET16b plasmid. (I) Induced cells with Astrosclerin-1 pET16b plasmid. (J) Purified inclusion bodies consist of insoluble Astrosclerin-1.

### Fig. 5

Phylogenetic analysis of the Astrosclerins and representative members of the α carbonic anhydrases (α-CAs). (A) Bayesian and Maximum Likelikihood methods were used to reconstruct the evolutionary history of the α-CAs, with a non-metazoan α-CA as an outgroup. The tree topology shown here is derived from a Bayesian analysis. Unlabelled nodes indicate 100 % support from both analyses. Experimentally validated sub-cellular localizations are indicated by solid colored lines. Predicted or inferred localizations are indicated by dashed lines. (B) An abbreviated protein alignment representing conserved blocks shared between the Astrosclerins, AmqCA1, the earliest branching vertebrate α-CA clade (α-CAIV), one of the most active α-CA isoenzymes known, α-CAII and the reconstructed ancestral metazoan (RAM) α-CA. Circles indicate putative active site residues, zinc coordinating Histidine residues and proton shuttling Histidine residue, respectively. The left-most box indicates the consensus motif returned by Edman sequencing.

### Detailed description of the present invention

The inventors have now identified new polypeptides having carbonic anhydrase activity in coralline sponges.

In describing the present invention, the following terms will be employed and are intended to be defined as indicated below.

As used herein, the expression "polypeptide" refers to any peptide or protein comprising two or more amino acids joined to each other by peptide bonds or modified peptide bonds. Polypeptide refers to both short chains, commonly referred to as peptides, polypeptides or oligomers, and to longer chains generally referred to as proteins. Polypeptides may contain amino acids other than the 20 gene-encoded amino acids. Polypeptides include those modified either by natural processes, such as processing and other post-translational modifications, but also by chemical modification techniques. Such modifications are well described in basic texts and in more detailed monographs, as well as in a voluminous research literature and they are well known to those of skilled in the art. It will be appreciated that the same type of modification may be present in the same or varying degree at several sides in a given polypeptide. Preferably, the polypeptide comprises five or more amino acids, for example 10 or more amino acids, like 20 or more amino acids in particular 30 or more amino acids, like 50, 70 or 100 amino acids.

As used herein, the expression "functional derivative" refers to a protein / peptide / polypeptide sequence that possesses a functional biological activity that is substantially similar to the biological activity of the whole protein / peptide / polypeptide sequence. That is, the functional derivative refers to a polypeptide having carbonic anhydrase activity able to catalyse the hydration of carbon dioxide. Said functional derivative of the polypeptide having carbonic anhydrase activity may or may not contain post-translational modifications such as covalently linked carbohydrates, if such modification is not necessary for the performance of a specific function. The term "functional derivative" is meant to encompass fragments or chemical derivatives. In this connection the term "chemical derivative" refers to polypeptides containing additional chemical moieties not normally part of a protein / peptide, said moieties being added by using techniques well known in the art.

The expression "carbonic anhydrase activity" refers to the catalytic activity of converting CO₂ into HCO₃⁻ and H⁺ or vice versa, thus, degrading carbon dioxide or synthesising carbon dioxide from H⁺ and CO₃⁻.

The term "nucleic acid" is used in the present invention refers to DNA (dioxide nucleic acid) or RNA (ribonucleic acid) and their single or double stranded polymers (polynucleotides). The term "DNA" includes cDNA. Unless limited, the term includes those nucleic acids having similar structure with the reference nucleic acid and those natural nucleic acid analogues already known and metabolised in a similar way as natural nucleic acids.

The sequence similarity as used herein refers to expressing sequence identity compared to the sequence shown in SEQ ID Nos. 2, 4 and 5, respectively. Sequence similarity is identical with the term "sequence homology". Preferably, the sequence similarity or homology is at least 50%, for example 60%, 70%, 80%, 90%, more preferred at least 92%, like 95%, 96%, 97%, 98% and particular referred 99%.

One can use programmes like Clustal program to compare amino acid sequences or the blastp program allowing comparing amino acid sequences and finding the optimal alignment. These programmes allow calculating amino acid similarity or homology for an optimal alignment. That is amino acid alignment in sequence "identity" and "similarity" are determined from an optimal global alignment between the two sequences being compared. The skilled person is well aware of suitable programs doing similarity or homology analysis.

In a first embodiment, the present invention concerns a method for in vitro or in vivo degradation of carbon dioxide and/or amorphous or crystalline silicon dioxide or silicones and other silicon (IV)- or metal (IV)-compounds as well as of mixed polymers of these compounds, wherein a polypeptide or a metal complex of a polypeptide is used for the degradation, characterised in that the polypeptide comprises a carbonic anhydrase activity and exhibits a sequence similarity of at least 50% to the sequence shown in SEQ ID Nos. 2, 4 and 5, respectively, and said peptide is able to degrade carbon dioxide and/or amorphous or crystalline silicon dioxide.

In a further aspect, the present invention relates to a method for the synthesis of carbon dioxides and/or amorphous or crystalline silicon dioxide, silicones and other silicon (IV)- or metal (IV)-compounds as well as of mixed polymers of these compounds, wherein a polypeptide or a metal complex of a polypeptide is used for the synthesis, characterised in that said polypeptide has a carbonic anhydrase activity and has at least 50% sequence similarity to the sequence shown in SEQ ID Nos. 2, 4 and 5, respectively, and said polypeptide is able to degrade carbon dioxide and/or amorphous or crystalline silicon dioxide.

In addition, another preferred embodiment pertains to a method to capture CO₂ in a CO₂ containing fluid, comprising the step of contacting said CO₂ containing fluid with a polypeptide or a metal complex of a polypeptide, wherein said polypeptide has a carbonic anhydrase activity and exhibits a sequence similarity of at least 50% to the sequence shown in SEQ ID Nos. 2, 4 and 5, respectively.

The CO₂ containing fluid may be a CO₂ containing liquid or a CO₂ containing gas. Particular preferred are CO₂ containing exhaust gases from combustion chambers in incinerators or electric power, thermo power, steel, cement, paper, glass, ceramic plants or oil refineries.

In another preferred embodiment the method to capture CO₂ in a CO₂ containing fluid is used to capture CO₂ in catalysts of vehicles.

Depending on the environmental conditions, the polypeptide according to the present invention allows to degrade carbon dioxide and/or amorphous or crystalline silicone dioxide or silicones and other silicon (IV)- or metal (IV)-compounds as well as mixed polymers of these compounds or to synthesise carbon dioxide and/or amorphous or crystalline silicon dioxide, silicones and other silicon (IV)- or metal (IV)-compounds as well as mixed polymers of these compounds.

Preferably, the carbonic acid produced by the carbonic anhydrase activity of said polypeptide is further processed, e.g. by forming of a salt of the carbonic acid, e.g. an alkaline metal bicarbonate or an earth alkaline metal bicarbonate, like sodium bicarbonate or calcium bicarbonate.

In a further aspect, the present invention relates to a polypeptide of a carbonic anhydrase from *Astrosclera willeyana* substantially identical with the sequence of SEQ ID Nos. 2, 4 and 5, respectively, or a polypeptide being homologous thereto, which amino acid sequence of said polypeptide exhibits a sequence similarity of at least 50% to the sequence shown in SEQ ID Nos. 2, 4 and 5, respectively, a metal complex of the polypeptide or a functional derivative thereof, while said polypeptide or functional derivative thereof are able to degrade carbon dioxide and/or amorphous or crystalline silicon dioxide.

In a preferred embodiment, the polypeptide is derived or identical to the polypeptide according to Seq. ID No. 5.

Preferably, the polypeptide comprises a sequence shown in SEQ ID Nos. 2, 4 and 5, respectively. Particularly preferred, the polypeptide comprises the sequence of amino acid (aa) 25 to amino acid 255 of SEQ ID No. 2 (SEQ ID No.8), SEQ ID No. aa 25 to aa 255 of Seq. ID No. 4 (SEQ ID No. 9) and aa 25 to aa 255 of Seq. ID No. 5 (SEQ ID No. 10), respectively.

In this connection, it's noted that the metal complex of said polypeptide preferably comprises a zinc metal polypeptide having carbonic anhydrase enzyme activity. The carbonic anhydrase constitutes a zinc metal enzyme that are involved in that reversible hydration of CO₂. Thus, the metal complex polypeptide is preferably a zinc metal complex of said polypeptide. Of course other metal ions may be present in the metal complex of said polypeptide as long as the carbonic anhydrase activity is maintained.

In a further embodiment, the present invention relates to an isolated polynucleotide encoding a polypeptide as defined above having a carbonic anhydrase activity. Said nucleic acid may be in form of a DNA, cDNA, RNA or mixtures thereof. Preferably, the isolated polynucleotide comprises a nucleic acid sequence which is substantially identical to the sequence shown in SEQ ID Nos. 1, 3 and 5, respectively, or a functional fragment thereof.

In this connection functional fragment as it is generally understood and used herein, refers to a nucleic acid sequence that encodes for a functional biological activity of protein that is substantially similar to the biological activity of protein coding of the whole nucleic acid sequence. In other words, it refers to a nucleic acid or fragment(s) thereof that substantially retains the capacity of encoding a carbonic anhydrase polypeptide of the invention.

By "substantially identical" when referring to a polynucleotide, it will be understood that the polynucleotide of the invention has a nucleic acid sequence which is at least 65% identical, more particularly 80% identical and even more particularly 95%, like 96 %, 97 %, 98 % or 99 % identical to the sequence of SEQ ID Nos. 1, 3 and 5, respectively, or functional fragments thereof.

Preferably, the nucleic acid is a sequence shown in Seq. ID No. 5 or a functional fragment thereof.

In a preferred embodiment the nucleic acid is in the form of the complementary antisense sequence, e.g. for medicinal purposes.

The present invention relates further to expression or cloning vectors containing the polynucleotides according to present invention.

As used herein, the term "vector" refers to a polynucleotide construct designed for transduction/transfection of one or more cell types. Vectors may be for example cloning vectors which are designed for isolation, propagation and replication of inserted nucleotides, expression vectors which are designed for transcription of a nucleotide sequence in a host cell or other vectors known in the art, e.g. viral vectors or shuttle vectors.

Moreover, the present invention pertains to host cells containing a vector according to the present invention or polynucleotides according to the present invention. Said host cells may be any type of cell like transiently transfected or permanently transfected mammalian cell lines, isolated primary cells or other eukaryotic or prokaryotic cell systems known in the art, e.g. insect cells, yeast systems, plant cells, bacterial systems etc.

Further, the present invention pertains to a pharmaceutical preparation containing a polypeptide according to present invention, polynucleotide molecules according to the present invention, vectors or host cells as defined herein. Said pharmaceutical preparations may be in a form known to the skilled person typically in combination with a pharmaceutically acceptable carrier system.

The pharmaceutical compositions of the inventions can be used for therapy, prophylaxis, treatment of a relapse of a disorder, diagnosis and prognosis. The pharmaceutical compositions can be administered orally, sublingually, injected intravenously, injected subcutaneously, injected into tissue, administered topically, inhaled as aerosol or administered rectal or through other routes. The pharmaceutical composition can be supplied as tablets, pills, capsules, powder, granulates, salves, plasters, releasing substances transdermal to the individual, tinctures, uvula, suspensions, solutions, etc. The pharmaceutical compositions can comprise additives such as filling additives, antimicrobial additives, preservative additives, colouring additives, flavouring additives, smelling additives, protective or stabilising additives etc. The pharmaceutical compositions can be applied to an individual need once or repeatedly. It can be applied to one or more times daily, weekly, monthly or in longer time intervals. The pharmaceutical compositions can be used alone or in combination with other pharmaceutical compositions. The exact dose of the active ingredient will depend on the purpose of the treatment, and will be ascertainable by one skilled in the art using known techniques. As it is known in the art, adjustments for systemic versus localised delivery, age, general health, sex, diet, time of administration, drug interaction and the severity of the condition may be necessary, and will be ascertainable with routine experimentation by those skilled in the art.

In particular, antisense polynucleotides may be used for treating various diseases involving carbonic anhydrase activity, e.g. glaucoma treatment, cancer treatment, etc.

Finally, the present invention relates to methods for identifying inhibitors or activators of the polypeptide according to the present invention having a carbonic anhydrase activity from *Astrosclera willeyana* according to SEQ ID Nos. 2, 4 and 5, respectively, or a polypeptide being homologous thereto having a sequence similarity of at least 50% to the sequence shown in SEQ ID Nos. 2, 4 and 5, respectively, or a functional fragment thereof comprising the steps of i) providing said carbonic anhydrase, ii) contacting said carbonic anhydrase with a potential inhibitor or activator and iii) measuring the ability of a polypeptide to degrade or synthesise carbon dioxide and/or amorphous or crystalline silicon dioxide, silicones and other silicon (IV)- or metal (IV)-compounds as well as mixed polymers of these compounds. Preferably, the polypeptide is provided in vivo, e.g. in a living cell system, in a cellular extract or lysate or in purified form.

Silicones are important technical materials. However, various silicon compounds can only be produced in a cost intensive manner or at present only in small amounts as mineral resources, respectively, and can therefore only be isolated with considerable effort. The process of the chemical synthesis of silicates requires drastic conditions, such as high pressure and high temperature.

In contrast, with the aid of specific enzymes, e.g. derived from sponges and algae, it is possible to form silicate scaffolds under natural conditions with advantages regarding specificity, coordinated information, adjustability and the possibility for synthesising nanostructures. In this connection it is noted that silicon dioxide can be found both in crystallised and amorphous forms, e.g. the shells of diatoms and the spicules of diatomeus sponges consist of amorphous SiO₂. Silicones or siloxanes are generated by a partial replacement of the OH-group in the silicic acid by single bond organylic residues that do not participate in the condensation process. The silicon dioxide comprises condensation products of the silicic acid, silicates.

Preferred is a method according to the present invention that is characterised in that compounds such as silicic acids, monoalkoxy silantrioles, dialkoxy silandioles, trialkoxy silnanoles, tetrakoxy silanes, alkyl- or aryl-silant-rioles, alkyl- or aryl-monoalkoxy silandioles, alkyl- or aryl-diakoxy silanoles, alkyl- or aryl-trialkoxy silanes or other metal(IV)-compounds are used as reactants (substrates) for the synthesis. By using defined mixtures of the compounds mixed polymers having a defined composition can be produced.

According to a further aspect of the present invention a formation of defined two- and three-dimensional structures can occur by the polypeptide or a metal complex of the polypeptide or the binding of the polypeptide or a metal complexes of the polypeptide to other molecules or the surfaces of glass, metals, metal oxides, plastics, biopolymers or other materials as a template.

The polypeptide according to the invention can be characterised in that the polypeptide has been synthetically produced or that the polypeptide or the metal complex of the polypeptide is present in a prokaryotic or eukaryotic cell extract or lysate. The cell extract or lysate can be obtained from a cell ex vivo or ex vitro, for example for a recombinant bacterial cell or a marine sponge.

### Examples

The present invention will be more readily understood by referring to the following examples. These examples are illustrative of the wide range of applicability of the present invention are not intended to limit their scope. Modifications and variations can be made therein.

### Protein sequencing and full length cDNA

Specimens of *A. willeyana* were collected around Lizard Island and Ribbon Reef #10 (Great Barrier Reef, Australia) from subtidal reef caves by SCUBA diving. Specimens were prepared for Scanning and Transmission Electron Microscopy (SEM, TEM) according to G. Wörheide, Facies 38, 1 (1998). Specimens for biochemical analysis were processed as described in G. Wörheide, L. Macis, D. J. Jackson, J. Reitner, in Proceedings of the 9th International Symposium on Biomineralization, Pucon, Chile. Briefly, samples were frozen and subsequently freeze-dried and the tissue removed with 2 % sodium hypochlorite. The aragonitic skeleton was crushed in a rock mill and then decalcified with acetic acid (pH 4.0) overnight or until the pH stabilized at 4.0 and the skeleton had completely dissolved. This solution, containing soluble matrix proteins, was centrifuged for 5 minutes at 3000 rpm, desalted using a PD10 column (Pharmacia), eluted with sodium chloride (6g/1) and concentrated by ultrafiltration using a 30 kD column (Pall). Proteins were subsequently analyzed by SDS-PAGE.

Gels (14 %) were stained using a Silver-Stain Kit (Amersham Biosciences). Proteins were blotted onto PVDF membranes, stained with Coomassie Blue, and were subjected to Edman degradation on a Procise CLC sequenator (Applied Biosystems) at the Institute for Biochemistry II of the University of Göttingen. Three prominent bands (33 - 41 kDa) were subjected to N-terminal Edman sequencing. A shared sequence motif of DFNYY (SEQ ID No. 14) was identified and used to design degenerate PCR primers for 5' and 3' RACE PCR. A cDNA RACE library was constructed with a BD Biosciences SMART RACE kit, and from RNA isolated from an entire adult individual using a QIAGEN RNeasy plant mini kit. RACE fragments were cloned into a pCR4 TOPO vector and sequenced using standard BigDye Terminator (version 3.1) methods. Sequences were aligned in CodonCode Aligner (version 1.6.3) and full length cDNA contigs generated. Full length cDNA sequences are deposited under GenBank accession numbers EF434876, EF434877 and EF434878 for Astrosclerin-1, -2 and -3 respectively. Primers that would amplify both the open reading frame (for expression vector construction and the majority of the primary transcripts (for riboprobe construction and partial intron mapping) were then designed. Introns were partially characterized by amplifying the *Astrosclerins* from genomic DNA.

### Primary sequence analysis and phylogenetic inference

The presence of putative sequences in derived protein sequences were detected using the SignalP server (J. Dyrlov Bendtsen, H. Nielsen, G. von Heijne, S. Brunak, J. Mol. Biol. 340, 783 (2004)). A set of α-carbonic anhydrase representative of the major subfamilies was downloaded from GenBank and α-CA homologs from the demosponge *Amphimedon queenslandica* and the starlet anemone *Nematostella vectensis* were identified using publicly available genomic traces. Genomic contigs were assembled using in house assembly software and submitted to Genbank under accession numbers EF434873, EF434874 and EF434875 for *AmqCA1, AmqCA2* and *AmqCA3* and EF434879 for *NvecCA1.* EST and genome trace alignments for AmqCA1 spanning the 295 residue ORF were unambiguous. The α-CA protein dataset was aligned using ClustalX (multiple alignment parameters: gap opening 3.0; gap extension 1.8). The resulting alignment was used for phylogenetic analyses using Bayesian and maximum likelihood (ML) methods. Appropriate models of protein evolution were assessed using Protest (F. Abascal, R. Zardoya, D. Posada, Bioinformatics 21, 2104 (2005)). Bayesian analyses were carried out with the parallel version of MrBayes (v. 3.1.2) on a Linux cluster at the Gesellschaft für wissenschaftliche Datenverarbeitung Göttingen (GWDG; www.gwdg.de). The following settings were used: prset aamodelpr=fixed(wag); nchains=8 nruns_2 temp=0.2 printfreq=1000 samplefreq=100. Analyses were run for 15 million generations and adequate convergence of chains was confirmed using AWTY (J.C. Wilgenbusch, D.L. Warren, D.L. Swofford (2004)). 1000 bootstrap replicates were achieved by running two analyses of 500 bootstraps.

Ancestral sequence reconstructions were performed with ANCESCON (W. Cai, J. Pei, N. Grishin, BMC Evolutionary Biology 4, 33 (2004) using the "marginal" and "joint" methods. The rate factor was set to ML, the gap content for rate factor calculation was set to 0.5, and the PI vector was optimized for each site with the Powell method.

### Vector design, recombinant expression and purification of Astrosclerin-1, -2 and -3

The cDNA sequence encoding the ORF Astrosclerin-1, -2 and -3 (sequences encoding signed peptides were omitted) were amplified from cDNA using forward (5'-CCGCCATGGGTCGGTGTGATTTCAACTACTACAATC-3') (SEQ ID No. 11) and reverse (5'-CCGCCATGGTCAGCCATGATGGTCTCTGCCGTGACG-3') (SEQ ID No. 12) primers with *Ncol* restriction sites (underlined) to facilitate cloning into the pET16b expression vector (Novagen). These primers resulted in the incorporation of Met and Gly residues at the amino terminus of the mature protein, and eliminated the carboxy His tag sequence. Plasmids containing inserts in the correct orientation were sequenced to confirm ORF fidelity. The *E. coli* host strain B(DE3) from Novagen was transformed via heat shock, and colonies were selected on LB agar plates supplemented with Ampicilin (50 µg/ml), Kanamycin (50 µg/ml) and Tetracycline (12.5 µg/ml). Well grown single colonies were used to innoculate LB media with the above concentrations of antibiotics, and grown at 37 °C overnight in a 10 ml volume. This was used to innoculate 500 ml of LB media containing the appropriate antibiotics and grown at 37 °C until the OD₆₀₀ was 0.8. Expression of recombinant Astrosclerin was induced by adding IPTG to a final concentration of 1 mM and continuing incubation at 37 °C for a further 4 hours. Bacterial cells were collected by centrifugation at 2800 x g in an Eppendorf 5403 centrifuge for 15 min. at 4 °C. The pelleted cells were stored at -80 °C until further use. Lysis was achieved in a modified RIPA buffer (25 mM Tris pH 8.5, 150 mM NaCl, 1% Triton, 1% w/v deoxycholic acid, 1 mg/ml lysozyme and 1 mM PMSF) with 10 ml of buffer per gram of cell pellet. Cells were suspended in RIPA and gently rotated at room temperature for 15 min. whereafter 3 freeze thaw cycles ensured thorough lysis. MgCl₂ and CaCl₂ were added to final concentrations of 25 mM and 5 mM respectively followed by 6 units of DNAse. This lysate was incubated at 37 °C for 15 min to digest genomic DNA and then spun at 14,000 x g for 15 min at 4 °C. The lysate then underwent a solvent exchange for binding buffer (25 mM Tris pH 8.3 and 250 mM Na₂SO₄ using a Nanosep 30 kD column (Pall Life Sciences OD030C33). This solution was then subjected to affinity purification using a HiTrap NHS-activated HP 1 ml column (GE Healthcare 17-0716-01) coupled with 4-Aminomethylbenzenesulfonamide hydrochloride (Sigma A2134) roughly following the protocol described by C. L. Bering, J. J. Kuhns, R. Rowlett, J. Chem. Edu. 75, 1021 (1998). Recombinant Astrosclerin was allowed to bind to the column for 1 hour at room temperature, which was then washed with 4 column volumes of binding buffer. Recombinant protein was eluted from the column with 0.4M KSCN, 25 mM Tris pH 8.3. Half column volumes (500 µl) were collected and the A280 absorbance measured. The majority of the protein was eluted from the column in fractions 3-4.

These were pooled, concentrated and the solvent exchanged for 25 mM Tris, 10 µM ZnSO₄ using a Nanosep 10 kD column (Pall Life Sciences OD010C33). The concentration of this solution was measured using an percent solution extinction coefficient of 1.141 that was determined by the ExPASy ProtParam tool (E. Gasteiger et al., Nucleic Acids Res. 31, 3784 (2003)).

### Expression and purification of Astroscierin-1

A second form of Astrosclerin here named Astrosclerin-1 was isolated by a degernate PCR approach and clonend into the pET16b expression vector using forward (5'-CCGCCATGGGTCGGTGTGATTTCAACTACTACAATC-3') (SEQ ID No. 11) and reverse (5'-CCGCCATGGGCGTTTGCGCTTCTTGCCTTCACC-3') (SEQ ID No. 13) primers.

Astrosclerin-1 was expressed and released from B(DE3) cells following the same methods as described above for Astrosclerin-3. Astrosclerin-1 was expressed as an insoluble protein. Inclusion bodies (IBs) were separated from the lysis supernatant by centrifugation at 11,000 rpm for 15 min. at 4 °C. The pelleted IBs were washed twice in a solution of 50 mM Tris pH 8.0, 150 mM NaCl, 1 mM EDTA and 1% Triton followed by two washes in 50 mM Tris pH 8.0, 150 mM NaCl to remove Triton and EDTA. The final pellet was weighed and dissolved in 10 ml of denaturation buffer (5M Guanidine hydrochloride, 50 mM Tris pH 8.5 100 mM NaCl and 10 mM DTT) per gram of IB pellet. The concentration of this protein solution was measured using the Bradford assay with BSA dissolved in denaturation buffer as a standard. The concentration of denatured protein was adjusted to 5 mg/ml with denaturation buffer. Refolding of Astrosclerin was attempted under a variety of conditions, but the following describes a procedure that recovered the highest possible concentration of Astrosclerin-1. 200 µl of the denatured 5 mg/ml solution was gradually added to 800 µl of refolding buffer (1 M L-arginine, 50 mM Tris pH 8.5, 0.5 mM oxidised glutathione, 5.0 mM reduced glutathione, 1 mM PMSF) resulting a final concentration of 1 mg/ml protein and 1 M Guanidine hydrochloride. Refolding was allowed to proceed for 12 hours at room temperature prior to assaying for activity. This regime was selected on the basis of previous refolding studies of carbonic anhydrase (D. B. Wetlaufer, Y. Xie, Protein Sci. 4 1535 (1995), Y. Xie, D. B. Wetlaufer, Protein Sci. 5, 517 (1996), N. Armostron, L. de, A, E. Gouaux, Protein Sci. 8, 1475 (1999)).

### Electrometric assay for Astrosclerin-1 and -3

Activity of recombinant Astrosclerin isoenzymes 1 and 3 was quantified using a modification of the Wilbur-Anderson assay (K. M. Wilbur, N. G. Anderson, J. Biol. Chem. 176, 147 (1948)). 1 µg of recombinant protein was added to 2 ml of ice cold 25 mM Tris pH 8.3 with 10 µM ZnS0₄. 1 ml of freshly prepared CO₂ saturated water (prepared by passing CO₂ through ice cold MiliQ water for 45 minutes) was then quickly added and the time taken for the pH to fall from 8.3 to 6.3 was monitored using a 540 GLP pH meter connected to a workstation running Multi/ACAHT II software. pH measurements were taken every second with the reaction performed on ice with constant stirring. Control reactions consisted of all reagents minus protein. The CA specific inhibitor Acetazolamide was dissolved in DMSO at a concentration of 1 mM and added to the assay at a final concentration of 1 µM. DMSO control reactions indicated that this solvent did not affect the reaction (data not shown). Units of activity were calculated using the equation U/mg = (to - t)/t where t₀ is the time taken for the pH to drop from 8.3 to 6.3 in an uncatalyzed control reaction, and t is the time taken for the same pH drop in a catalyzed reaction.

### Determination of activity for degrading silicon dioxide

As substrate (amorphous silicone dioxide) for the silicon dioxide degrading activity, e.g. spicules of S. *domuncula* are suitable. The spicules can be obtained from sponge tissue by 12-hour incubation in the presence of ethylene diamine tetraacetec acid (20 mM, in PBS=phosphate buffer-salt-solution, consisting of 1.15 mM KH₂PO₄, 8.1 mM Na₂POO₄, 137 mM NaCl and 2.7 mM KCl). After washing with destilled water and with ethanol (two times) the spicules are dried (56°C.) and then grinded to a powder in a mortar.

The activity for degrading silicon dioxide can be determined as follows. Commonly, 100µg of the dried spicules (powder) are added to a suitable buffer, such as 50 mM Tris-HCl-buffer (pH 7.2; 10 mM DL-dithiothreithol, 100 mM NaCl) and 0.5 mM ZnSo₄ in 2 ml Eppendorf-tubes. Then, usually 50 µl of the recombinant enzyme are added, and incubated at 25°C (the incubation is possible also at other temperatures between 5°C and about 65°C). The average incubation time is 60 minutes. For a quantitative determination of the amount of dissolved silicon dioxide, the non dissolved spicules are spun off (14000xg; 15 minutes; 4°C). The released soluble silicic acid can be quantitatively determined e.g. with the aid of a molybdenum-supported determination methods such as e.g. the colorimetric "Silicon Test" (Merck, 1.14794). In this case, the amount of silicic acid is calculated from the values of extinction at 810 nm based on a calibration curve with a silicon standard (Merck 1.09947).

## Claims

1. Method for in vitro or in vivo degradation of carbon dioxide and/or amorphous or crystalline silicon dioxide or silicones and other silicon (IV)- or metal (IV)-compounds as well as of mixed polymers of these compounds, wherein a polypeptide or a metal complex of a polypeptide is used for the degradation, **characterised in that** the polypeptide comprises a carbonic anhydrase activity and exhibits a sequence similarity of at least 50 % to the sequence shown in SEQ ID Nos. 2, 4 and 5, respectively, and said peptide is able to degrade carbon dioxide and/or amorphous or crystalline silicon dioxide.

2. Method for the synthesis of carbon dioxide and/or amorphous or crystalline silicon dioxide, silicones and other silicon (IV)-or metal (IV)-compounds as well as of mixed polymers of these compounds, wherein a polypeptide or a metal complex of a polypeptide is used for the synthesis, **characterised in that** said polypeptide has a carbonic anhydrase activity and has at least 50 % sequence similarity to the sequence shown in SEQ ID Nos. 2, 4 and 5, respectively, and said polypeptide is a able to degrade carbon dioxide and/or amorphous or crystalline silicon dioxide.

3. A method to capture CO₂ in a CO₂-containing fluid, particularly in an exhaust gas, comprising the step of contacting said CO₂-containing fluid with a polypeptide or a metal complex of a polypeptide, wherein said polypeptide has a carbonic anhydrase activity and exhibits a sequence similarity of at least 50 % to the sequence shown in SEQ ID Nos. 2, 4 and 5, respectively.

4. A polypeptide of a carbonic anhydrase from *Astrosclera willeyana* according to SEQ ID Nos. 2, 4 and 5, respectively, or a polypeptide being homologous thereto, which amino acid sequence of said polypeptide exhibits a sequence similarity of at least 50 % to the sequence shown in SEQ ID Nos. 2, 4 and 5, respectively, a metal complex of the polypeptide, or a functional derivative thereof.

5. The polypeptide according to claim 4, comprising the sequence shown in SEQ ID Nos. 2, 4 or 5.

6. An isolated polynucleotide encoding a polypeptide as defined in claims 4 or 5.

7. The isolated polynucleotide of claim 6, comprising a nucleic acid sequence which is a substantially identical to the sequence shown in SEQ ID Nos. 1, 3 or 5 or a functional fragment thereof.

8. The polynucleotide according to claim 6 or 7, **characterised in that** it is present in a form of DNA, cDNA, RNA or mixtures thereof.

9. The polynucleotide according to anyone of claims 5 to 7 in the form of its complementary "antisense"-sequence.

10. An expression or cloning vector, containing a nucleic acid according to anyone of claims 6 to 9.

11. A host cell comprising a vector as defined in claim 11 or a nucleic acid according to anyone of claims 6 to 9.

12. Pharmaceutical preparation contain a polypeptide according to claim 4 or 5 or polynucleotide molecules according to claims 5 to 9, a vector according to claim 10 and/or host cells according to claim 11.

13. A method for identifying inhibitors or activators of a polypeptide of a carbonic anhydrase from *Astrosclera willeyana* according to SEQ ID Nos. 2, 4 and 5, respectively, or a polypeptide being homologous thereto having a sequence similarity of at least 50 % to the sequence shown in SEQ ID Nos. 2, 4 and 5, respectively, or a functional fragment thereof comprising the step of providing said carbonic anhydrase and contacting said carbonic anhydrase with a potential inhibitor or activator and measuring the ability of a polypeptide to degrade or synthesise carbon dioxide and/or amorphous or crystalline silicon dioxide, silicones and other silicon (IV)- or metal (IV)-compounds as well as of mixed polymers of theses compounds.

14. The method according to claim 13 wherein said polypeptide is provided in vivo, in a cellular extract or lysate or purified form.


SEQUENCE LISTING

<110>  Georg-August-Universität Göttingen

<120>  Carbonic anhydrase and methods using the same

<130>  3294-037_EP

<150>  07008083.3
<151>  2007-04-20

<150>  EP07008083.3
<151>  2007-04-20

<160>  14

<170>  PatentIn version 3.3

<210>  1
<211>  1208
<212>  DNA
<213>  Astrosclera willeyana

<220>
<221>  CDS
<222>  (64)..(897)

<400>  1
gcggggaccc tggaggacac ttgaactwaa cctggagcta taactttagt taccagtaac      60

aga atg atg gca tac aaa gtt ctc cac ttc gta gtg att agt ctg gga      108
    Met Met Ala Tyr Lys Val Leu His Phe Val Val Ile Ser Leu Gly
    1               5                   10                  15

ctg gtt aca ttg gtt gcc agt cgg tgt gat ttc aac tac tac aat caa      156
Leu Val Thr Leu Val Ala Ser Arg Cys Asp Phe Asn Tyr Tyr Asn Gln
                20                  25                  30

cgt gca tgg ctg tcc tgc cca gga tca cag tgt ggt gga aat cga cag      204
Arg Ala Trp Leu Ser Cys Pro Gly Ser Gln Cys Gly Gly Asn Arg Gln
            35                  40                  45

tcc cca atc aac atc gat act agg gat aca aag gca aat aac aac ctc      252
Ser Pro Ile Asn Ile Asp Thr Arg Asp Thr Lys Ala Asn Asn Asn Leu
        50                  55                  60

att gct cta cga ttc aac aac tac aaa gtt cca gtg gat gga gat ttt      300
Ile Ala Leu Arg Phe Asn Asn Tyr Lys Val Pro Val Asp Gly Asp Phe
    65                  70                  75

gag aac ttg ggc aca gat gta gag ttt gtt cct aaa aac aag cat gcc      348
Glu Asn Leu Gly Thr Asp Val Glu Phe Val Pro Lys Asn Lys His Ala
80                  85                  90                  95

acc tta act aat cac cta gga acg tat gtt ctg cag caa ttt cag ttc      396
Thr Leu Thr Asn His Leu Gly Thr Tyr Val Leu Gln Gln Phe Gln Phe
                100                 105                 110

cac tgg gga agg gat tca tct gaa gga tcg gaa cac cga att gat ggc      444
His Trp Gly Arg Asp Ser Ser Glu Gly Ser Glu His Arg Ile Asp Gly
            115                 120                 125

caa caa tat gct gct gag att cac ttt gtt cac ctc aag caa ggt gca      492
Gln Gln Tyr Ala Ala Glu Ile His Phe Val His Leu Lys Gln Gly Ala
        130                 135                 140

tct caa aat ggt act gct ggt gat acc tat tct gta gtt gct gtg ctt      540

Ser Gln Asn Gly Thr Ala Gly Asp Thr Tyr Ser Val Val Ala Val Leu
    145                 150                 155

tgt gaa gct gca gac att cca ata aga gga gtg tgg cgt aac ctt agc      588
Cys Glu Ala Ala Asp Ile Pro Ile Arg Gly Val Trp Arg Asn Leu Ser
160                 165                 170                 175

cct gtt cca act gta cat aaa gac tcc cat tgt atc tta gat ctt gta      636
Pro Val Pro Thr Val His Lys Asp Ser His Cys Ile Leu Asp Leu Val
                180                 185                 190

tat gct gat ctc ctc cca cga aat cga gat tac tac cac tat gaa gga      684
Tyr Ala Asp Leu Leu Pro Arg Asn Arg Asp Tyr Tyr His Tyr Glu Gly
            195                 200                 205

tct ctc aca act cca ctc tgt gat gag act gtc cag tgg ttt gtg ctg      732
Ser Leu Thr Thr Pro Leu Cys Asp Glu Thr Val Gln Trp Phe Val Leu
        210                 215                 220

aag aat acc atc aaa att cct gag gcg ttt ctg acc agg ttg aga agg      780
Lys Asn Thr Ile Lys Ile Pro Glu Ala Phe Leu Thr Arg Leu Arg Arg
    225                 230                 235

gtg gag agg cat cgg agt ggt aca ctc ctc acc ttc aac ttc cgt aaa      828
Val Glu Arg His Arg Ser Gly Thr Leu Leu Thr Phe Asn Phe Arg Lys
240                 245                 250                 255

tta cag agg ctg cat ggt cgg gat gtc ttt gaa ttc cct ttc att ggt      876
Leu Gln Arg Leu His Gly Arg Asp Val Phe Glu Phe Pro Phe Ile Gly
                260                 265                 270

gaa ggc aag aag cgc aaa cgc tgaaacaact gtcatggcag acaccatcat      927
Glu Gly Lys Lys Arg Lys Arg
            275

ggctgatgtc acaatggaaa taatgaacat ttcaatttgg atttggaaac ttgttgtttc      987

ataatctagt tcagtctaaa actgctatga gacatttacc aatgtcagta gtttagttag     1047

ctaacttttg tactagctga atgattgtcc atagtttctg tagtctgtta aaaacctctc     1107

agatggaact ggtctcaaac tgaatattat tctgcttgag accattgatt ttgttgctgg     1167

ttaatgacat tacgtgcaaa aaaaaaaaaa aaaaaaaaaa a                         1208

<210>  2
<211>  278
<212>  PRT
<213>  Astrosclera willeyana

<400>  2

Met Met Ala Tyr Lys Val Leu His Phe Val Val Ile Ser Leu Gly Leu
1               5                   10                  15

Val Thr Leu Val Ala Ser Arg Cys Asp Phe Asn Tyr Tyr Asn Gln Arg
            20                  25                  30

Ala Trp Leu Ser Cys Pro Gly Ser Gln Cys Gly Gly Asn Arg Gln Ser
        35                  40                  45

Pro Ile Asn Ile Asp Thr Arg Asp Thr Lys Ala Asn Asn Asn Leu Ile
    50                  55                  60

Ala Leu Arg Phe Asn Asn Tyr Lys Val Pro Val Asp Gly Asp Phe Glu
65                  70                  75                  80

Asn Leu Gly Thr Asp Val Glu Phe Val Pro Lys Asn Lys His Ala Thr
                85                  90                  95

Leu Thr Asn His Leu Gly Thr Tyr Val Leu Gln Gln Phe Gln Phe His
            100                 105                 110

Trp Gly Arg Asp Ser Ser Glu Gly Ser Glu His Arg Ile Asp Gly Gln
        115                 120                 125

Gln Tyr Ala Ala Glu Ile His Phe Val His Leu Lys Gln Gly Ala Ser
    130                 135                 140

Gln Asn Gly Thr Ala Gly Asp Thr Tyr Ser Val Val Ala Val Leu Cys
145                 150                 155                 160

Glu Ala Ala Asp Ile Pro Ile Arg Gly Val Trp Arg Asn Leu Ser Pro
                165                 170                 175

Val Pro Thr Val His Lys Asp Ser His Cys Ile Leu Asp Leu Val Tyr
            180                 185                 190

Ala Asp Leu Leu Pro Arg Asn Arg Asp Tyr Tyr His Tyr Glu Gly Ser
        195                 200                 205

Leu Thr Thr Pro Leu Cys Asp Glu Thr Val Gln Trp Phe Val Leu Lys
    210                 215                 220

Asn Thr Ile Lys Ile Pro Glu Ala Phe Leu Thr Arg Leu Arg Arg Val
225                 230                 235                 240

Glu Arg His Arg Ser Gly Thr Leu Leu Thr Phe Asn Phe Arg Lys Leu
                245                 250                 255

Gln Arg Leu His Gly Arg Asp Val Phe Glu Phe Pro Phe Ile Gly Glu
            260                 265                 270

Gly Lys Lys Arg Lys Arg
        275

<210>  3
<211>  1213
<212>  DNA
<213>  Astroslcera willeyana

<220>
<221>  CDS
<222>  (59)..(934)

<400>  3

gagcctggag gacacttgaa cttaacctgg agctataact ttacttacca gtaacaga 58

atg atg gca tac aaa gtt ctc cgc ttc gta gtg att agt ctg gga ctg 106
Met Met Ala Tyr Lys Val Leu Arg Phe Val Val Ile Ser Leu Gly Leu
1               5                   10                  15

gtt aca ttg gtt gcc agt cgg tgt gat ttc aac tac tac aat caa cat 154
Val Thr Leu Val Ala Ser Arg Cys Asp Phe Asn Tyr Tyr Asn Gln His
            20                  25                  30

gca tgg ctg ctg tgc tca gaa tca cag tgt ggt gga aat cga cag tcc 202
Ala Trp Leu Leu Cys Ser Glu Ser Gln Cys Gly Gly Asn Arg Gln Ser
        35                  40                  45

cca atc aac atc gat act gaa aaa aca aag gca aat aac aac ctc att 250
Pro Ile Asn Ile Asp Thr Glu Lys Thr Lys Ala Asn Asn Asn Leu Ile
    50                  55                  60

gct cta cgg ttc aac aac tac gat gat cca gtg gat gga gat ttt gag 298
Ala Leu Arg Phe Asn Asn Tyr Asp Asp Pro Val Asp Gly Asp Phe Glu
65                  70                  75                  80

aac gtg ggc acc act gta gag ttc gtt cct gaa aca aag gat gcc acc 346
Asn Val Gly Thr Thr Val Glu Phe Val Pro Glu Thr Lys Asp Ala Thr
                85                  90                  95

tta act aat cac cta gga acg tat gat ctg ctg cag ttt cac ttc cac 394
Leu Thr Asn His Leu Gly Thr Tyr Asp Leu Leu Gln Phe His Phe His
            100                 105                 110

tgg gga aga gat tca tct gaa gga tcg gag cac caa att gat gac gaa 442
Trp Gly Arg Asp Ser Ser Glu Gly Ser Glu His Gln Ile Asp Asp Glu
        115                 120                 125

caa tat tct gct gag atc caa ttt gtt cac ctc aag caa ggt gca tct 490
Gln Tyr Ser Ala Glu Ile Gln Phe Val His Leu Lys Gln Gly Ala Ser
    130                 135                 140

cct agt gat act gct ggt gat acc ttt tct gta gtt gct gta ctt tgt 538
Pro Ser Asp Thr Ala Gly Asp Thr Phe Ser Val Val Ala Val Leu Cys
145                 150                 155                 160

gaa gct gca gac att cca ata aga gga gtg tgg gct aaa ctt agc cct 586
Glu Ala Ala Asp Ile Pro Ile Arg Gly Val Trp Ala Lys Leu Ser Pro
                165                 170                 175

gtt cca act aaa cat gaa gac tcc cat tct gtc tca gat ctt gta tac 634
Val Pro Thr Lys His Glu Asp Ser His Ser Val Ser Asp Leu Val Tyr
            180                 185                 190

act gat ctc ctc cca cga aat cga gac tac tac cac tat gaa gga tct 682
Thr Asp Leu Leu Pro Arg Asn Arg Asp Tyr Tyr His Tyr Glu Gly Ser
        195                 200                 205

ctc aca act cca ctc tgc gat gag act gtc cag tgg ttt gtg ctg aag 730
Leu Thr Thr Pro Leu Cys Asp Glu Thr Val Gln Trp Phe Val Leu Lys
    210                 215                 220

aat acc atc aaa att ccc aag gcc ttt ctg acc atg ttg aga agg gtg 778
Asn Thr Ile Lys Ile Pro Lys Ala Phe Leu Thr Met Leu Arg Arg Val
225                 230                 235                 240

gag agt gat gag gat ggt aca ctc cta act ttc aac ttc cgt aac cta 826
Glu Ser Asp Glu Asp Gly Thr Leu Leu Thr Phe Asn Phe Arg Asn Leu
                245                 250                 255

cag agg ctg aac ggt cga caa gtc ttt gaa ttc cct cca gat gtt gat 874
Gln Arg Leu Asn Gly Arg Gln Val Phe Glu Phe Pro Pro Asp Val Asp

            260                 265                 270

agt ggc gaa gac aag aag cgc aaa cgc aga aac aac cat cgc ggc aga 922
Ser Gly Glu Asp Lys Lys Arg Lys Arg Arg Asn Asn His Arg Gly Arg
        275                 280                 285

cac cat cat ggc tgatgtcaca atggaaataa tgaacatttc aatttggatt 974
His His His Gly
    290

tggaaacttg tttcttagta tagctcagtc taaaactgct ttgagacatt taccaatgtt 1034

ggtagtttag ttagctaacc tttgtactag ttgaatgatt gtccatagtg tcattttttt 1094

tgtagtctgc taaaaacctc tcagatggaa gtggtctcaa actgaatatt attctgtttg 1154

agaccattaa ttttgttgct tgttaatgac taaaaaaaaa aaaaaaaaaa aaaaaaaaa 1213

<210> 4
<211> 292
<212> PRT
<213> Astroslcera willeyana

<400> 4

Met Met Ala Tyr Lys Val Leu Arg Phe Val Val Ile Ser Leu Gly Leu
1               5                   10                  15

Val Thr Leu Val Ala Ser Arg Cys Asp Phe Asn Tyr Tyr Asn Gln His
            20                  25                  30

Ala Trp Leu Leu Cys Ser Glu Ser Gln Cys Gly Gly Asn Arg Gln Ser
        35                  40                  45

Pro Ile Asn Ile Asp Thr Glu Lys Thr Lys Ala Asn Asn Asn Leu Ile
    50                  55                  60

Ala Leu Arg Phe Asn Asn Tyr Asp Asp Pro Val Asp Gly Asp Phe Glu
65                  70                  75                  80

Asn Val Gly Thr Thr Val Glu Phe Val Pro Glu Thr Lys Asp Ala Thr
                85                  90                  95

Leu Thr Asn His Leu Gly Thr Tyr Asp Leu Leu Gln Phe His Phe His
            100                 105                 110

Trp Gly Arg Asp Ser Ser Glu Gly Ser Glu His Gln Ile Asp Asp Glu
        115                 120                 125

Gln Tyr Ser Ala Glu Ile Gln Phe Val His Leu Lys Gln Gly Ala Ser
    130                 135                 140

Pro Ser Asp Thr Ala Gly Asp Thr Phe Ser Val Val Ala Val Leu Cys
145                 150                 155                 160

Glu Ala Ala Asp Ile Pro Ile Arg Gly Val Trp Ala Lys Leu Ser Pro
                165                 170                 175

Val Pro Thr Lys His Glu Asp Ser His Ser Val Ser Asp Leu Val Tyr
            180                 185                 190

Thr Asp Leu Leu Pro Arg Asn Arg Asp Tyr Tyr His Tyr Glu Gly Ser
        195                 200                 205

Leu Thr Thr Pro Leu Cys Asp Glu Thr Val Gln Trp Phe Val Leu Lys
    210                 215                 220

Asn Thr Ile Lys Ile Pro Lys Ala Phe Leu Thr Met Leu Arg Arg Val
225                 230                 235                 240

Glu Ser Asp Glu Asp Gly Thr Leu Leu Thr Phe Asn Phe Arg Asn Leu
                245                 250                 255

Gln Arg Leu Asn Gly Arg Gln Val Phe Glu Phe Pro Pro Asp Val Asp
            260                 265                 270

Ser Gly Glu Asp Lys Lys Arg Lys Arg Arg Asn Asn His Arg Gly Arg
        275                 280                 285

His His His Gly
    290

<210>  5
<211>  1339
<212>  DNA
<213>  Astrosclera willeyana

<220>
<221>  CDS
<222>  (58)..(75)

<220>
<221>  CDS
<222>  (194)..(1051)

<400>  5
accctggagg acacttgaac taaacctgga gctataactt tagttaccag taacaga       57

atg atg gca tac aaa ggt gagtatttat gctgattgta catggcagtt             105
Met Met Ala Tyr Lys Gly
1               5

taagcataaa agcaaattgc tagattgtat gcatatccaa atatacagtg tatttacata    165

attccatttg ataatgcaac ttgcagtt ctc cac ttc gta gtg att agt ctg       217
                               Leu His Phe Val Val Ile Ser Leu
                                                10

gga ctg gtt aca ttg gtt gcc agt cgg tgt gat ttc aac tac tac aat      265
Gly Leu Val Thr Leu Val Ala Ser Arg Cys Asp Phe Asn Tyr Tyr Asn
15                  20                  25                  30

caa cgt gca tgg ctg tcc tgc cca gga tca cag tgt ggt gga aat cga      313
Gln Arg Ala Trp Leu Ser Cys Pro Gly Ser Gln Cys Gly Gly Asn Arg
                35                  40                  45

cag tcc cca atc aac atc gat act gaa aaa aca aag gca aat aac agc 361
Gln Ser Pro Ile Asn Ile Asp Thr Glu Lys Thr Lys Ala Asn Asn Ser
            50                  55                  60

ctc att gct cta cgg ttc aac gac tac gat gat cca gtg gat gga gat 409
Leu Ile Ala Leu Arg Phe Asn Asp Tyr Asp Asp Pro Val Asp Gly Asp
        65                  70                  75

ttt gag aac ttg ggc acc act gta gag ttc gtt cct gaa aca aag gat 457
Phe Glu Asn Leu Gly Thr Thr Val Glu Phe Val Pro Glu Thr Lys Asp
    80                  85                  90

gcc acc tta act aat cac cta gga acg tat gat ctg ctg cag ttt cac 505
Ala Thr Leu Thr Asn His Leu Gly Thr Tyr Asp Leu Leu Gln Phe His
95                  100                 105                 110

ttc cac tgg gga aga gat tca tct gaa gga tcg gag cac cga att gat 553
Phe His Trp Gly Arg Asp Ser Ser Glu Gly Ser Glu His Arg Ile Asp
                115                 120                 125

gac gaa caa tat tct gct gag atc cac ttt gtt cac ctc aag caa ggt 601
Asp Glu Gln Tyr Ser Ala Glu Ile His Phe Val His Leu Lys Gln Gly
            130                 135                 140

gca tct cct agt gat act gct ggt gat acc ttt tct gta gtt gct gtg 649
Ala Ser Pro Ser Asp Thr Ala Gly Asp Thr Phe Ser Val Val Ala Val
        145                 150                 155

ctt tgt gaa gct gca gac att cca ata aga gga gtg tgg gct aaa ctt 697
Leu Cys Glu Ala Ala Asp Ile Pro Ile Arg Gly Val Trp Ala Lys Leu
    160                 165                 170

agc cct gtt cca act gga cat gaa gac tcc cat tct gtc tca gat ctt 745
Ser Pro Val Pro Thr Gly His Glu Asp Ser His Ser Val Ser Asp Leu
175                 180                 185                 190

gta tac act gat ctc ctc cca cga aat cga gac tac tac cac tat gaa 793
Val Tyr Thr Asp Leu Leu Pro Arg Asn Arg Asp Tyr Tyr His Tyr Glu
                195                 200                 205

gga tct ctc aca act cca ctc tgc gat gag act gtc cag tgg ttt gtg 841
Gly Ser Leu Thr Thr Pro Leu Cys Asp Glu Thr Val Gln Trp Phe Val
            210                 215                 220

ctg aag aat acc atc aaa att ccc aag gcc ttt ctg acc atg ttg aga 889
Leu Lys Asn Thr Ile Lys Ile Pro Lys Ala Phe Leu Thr Met Leu Arg
        225                 230                 235

agg gtg gag agt gat gag gat ggt aca ctc cta act ttc aac ttc cgt 937
Arg Val Glu Ser Asp Glu Asp Gly Thr Leu Leu Thr Phe Asn Phe Arg
    240                 245                 250

aac cta cag agg ctg aat ggt cga cag gtc ttt gaa ttc cct cca gat 985
Asn Leu Gln Arg Leu Asn Gly Arg Gln Val Phe Glu Phe Pro Pro Asp
255                 260                 265                 270

gtt gat aat ggc gaa gac aag aag cgc aaa cgc aga aac aac cgt cac 1033
Val Asp Asn Gly Glu Asp Lys Lys Arg Lys Arg Arg Asn Asn Arg His
                275                 280                 285

ggc aga gac cat cat ggc tgatgtcaca atggaaataa tgaacatttc 1081
Gly Arg Asp His His Gly
            290

aatttggatt tggaaacttg tttcatagta tagctcagtc taaaactgct ttgagacatt 1141

taccaatgtt ggtagttaag ttagctaacc tttgtactag ttgaatgatt gtccatagtg 1201

tcattttttt tgtagtctgc taaaaacctc tcagatggaa gtggtctcaa actgaatatt    1261

ctgtttgaga ccattaattt tgttgcttgt taatgacatt acatgcaaat ttaaaaaaaa    1321

aaaaaaaaaa aaaaaaaa                                                  1339

<210>  6
<211>  6
<212>  PRT
<213>  Astrosclera willeyana

<400>  6

Met Met Ala Tyr Lys Gly
1               5

<210>  7
<211>  286
<212>  PRT
<213>  Astrosclera willeyana

<400>  7

Leu His Phe Val Val Ile Ser Leu Gly Leu Val Thr Leu Val Ala Ser
1               5                   10                  15

Arg Cys Asp Phe Asn Tyr Tyr Asn Gln Arg Ala Trp Leu Ser Cys Pro
            20                  25                  30

Gly Ser Gln Cys Gly Gly Asn Arg Gln Ser Pro Ile Asn Ile Asp Thr
        35                  40                  45

Glu Lys Thr Lys Ala Asn Asn Ser Leu Ile Ala Leu Arg Phe Asn Asp
    50                  55                  60

Tyr Asp Asp Pro Val Asp Gly Asp Phe Glu Asn Leu Gly Thr Thr Val
65                  70                  75                  80

Glu Phe Val Pro Glu Thr Lys Asp Ala Thr Leu Thr Asn His Leu Gly
                85                  90                  95

Thr Tyr Asp Leu Leu Gln Phe His Phe His Trp Gly Arg Asp Ser Ser
            100                 105                 110

Glu Gly Ser Glu His Arg Ile Asp Asp Glu Gln Tyr Ser Ala Glu Ile
        115                 120                 125

His Phe Val His Leu Lys Gln Gly Ala Ser Pro Ser Asp Thr Ala Gly
    130                 135                 140

Asp Thr Phe Ser Val Val Ala Val Leu Cys Glu Ala Ala Asp Ile Pro
145                 150                 155                 160

Ile Arg Gly Val Trp Ala Lys Leu Ser Pro Val Pro Thr Gly His Glu
                165                 170                 175

Asp Ser His Ser Val Ser Asp Leu Val Tyr Thr Asp Leu Leu Pro Arg
            180                 185                 190

Asn Arg Asp Tyr Tyr His Tyr Glu Gly Ser Leu Thr Thr Pro Leu Cys
        195                 200                 205

Asp Glu Thr Val Gln Trp Phe Val Leu Lys Asn Thr Ile Lys Ile Pro
    210                 215                 220

Lys Ala Phe Leu Thr Met Leu Arg Arg Val Glu Ser Asp Glu Asp Gly
225                 230                 235                 240

Thr Leu Leu Thr Phe Asn Phe Arg Asn Leu Gln Arg Leu Asn Gly Arg
                245                 250                 255

Gln Val Phe Glu Phe Pro Pro Asp Val Asp Asn Gly Glu Asp Lys Lys
            260                 265                 270

Arg Lys Arg Arg Asn Asn Arg His Gly Arg Asp His His Gly
        275                 280                 285

<210> 8
<211> 231
<212> PRT
<213> astrosclera willeyana

<400> 8

Asp Phe Asn Tyr Tyr Asn Gln Arg Ala Trp Leu Ser Cys Pro Gly Ser
1               5                   10                  15

Gln Cys Gly Gly Asn Arg Gln Ser Pro Ile Asn Ile Asp Thr Arg Asp
            20                  25                  30

Thr Lys Ala Asn Asn Asn Leu Ile Ala Leu Arg Phe Asn Asn Tyr Lys
        35                  40                  45

Val Pro Val Asp Gly Asp Phe Glu Asn Leu Gly Thr Asp Val Glu Phe
    50                  55                  60

Val Pro Lys Asn Lys His Ala Thr Leu Thr Asn His Leu Gly Thr Tyr
65                  70                  75                  80

Val Leu Gln Gln Phe Gln Phe His Trp Gly Arg Asp Ser Ser Glu Gly
                85                  90                  95

Ser Glu His Arg Ile Asp Gly Gln Gln Tyr Ala Ala Glu Ile His Phe
            100                 105                 110

Val His Leu Lys Gln Gly Ala Ser Gln Asn Gly Thr Ala Gly Asp Thr
        115                 120                 125

Tyr Ser Val Val Ala Val Leu Cys Glu Ala Ala Asp Ile Pro Ile Arg
    130                 135                 140

Gly Val Trp Arg Asn Leu Ser Pro Val Pro Thr Val His Lys Asp Ser
145                 150                 155                 160

His Cys Ile Leu Asp Leu Val Tyr Ala Asp Leu Leu Pro Arg Asn Arg
                165                 170                 175

Asp Tyr Tyr His Tyr Glu Gly Ser Leu Thr Thr Pro Leu Cys Asp Glu
            180                 185                 190

Thr Val Gln Trp Phe Val Leu Lys Asn Thr Ile Lys Ile Pro Glu Ala
        195                 200                 205

Phe Leu Thr Arg Leu Arg Arg Val Glu Arg His Arg Ser Gly Thr Leu
    210                 215                 220

Leu Thr Phe Asn Phe Arg Lys
225                 230

<210>  9
<211>  231
<212>  PRT
<213>  astrosclera willeyana

<400>  9

Asp Phe Asn Tyr Tyr Asn Gln His Ala Trp Leu Leu Cys Ser Glu Ser
1               5                   10                  15

Gln Cys Gly Gly Asn Arg Gln Ser Pro Ile Asn Ile Asp Thr Glu Lys
            20                  25                  30

Thr Lys Ala Asn Asn Asn Leu Ile Ala Leu Arg Phe Asn Asn Tyr Asp
        35                  40                  45

Asp Pro Val Asp Gly Asp Phe Glu Asn Val Gly Thr Thr Val Glu Phe
    50                  55                  60

Val Pro Glu Thr Lys Asp Ala Thr Leu Thr Asn His Leu Gly Thr Tyr
65                  70                  75                  80

Asp Leu Leu Gln Phe His Phe His Trp Gly Arg Asp Ser Ser Glu Gly
                85                  90                  95

Ser Glu His Gln Ile Asp Asp Glu Gln Tyr Ser Ala Glu Ile Gln Phe
            100                 105                 110

Val His Leu Lys Gln Gly Ala Ser Pro Ser Asp Thr Ala Gly Asp Thr
        115                 120                 125

Phe Ser Val Val Ala Val Leu Cys Glu Ala Ala Asp Ile Pro Ile Arg
    130                 135                 140

Gly Val Trp Ala Lys Leu Ser Pro Val Pro Thr Lys His Glu Asp Ser
145                 150                 155                 160

His Ser Val Ser Asp Leu Val Tyr Thr Asp Leu Leu Pro Arg Asn Arg
                165                 170                 175

Asp Tyr Tyr His Tyr Glu Gly Ser Leu Thr Thr Pro Leu Cys Asp Glu
            180                 185                 190

Thr Val Gln Trp Phe Val Leu Lys Asn Thr Ile Lys Ile Pro Lys Ala
        195                 200                 205

Phe Leu Thr Met Leu Arg Arg Val Glu Ser Asp Glu Asp Gly Thr Leu
    210                 215                 220

Leu Thr Phe Asn Phe Arg Asn
225                 230

<210>  10
<211>  231
<212>  PRT
<213>  astrosclera willeyana

<400>  10

Asp Phe Asn Tyr Tyr Asn Gln Arg Ala Trp Leu Ser Cys Pro Gly Ser
1               5                   10                  15

Gln Cys Gly Gly Asn Arg Gln Ser Pro Ile Asn Ile Asp Thr Glu Lys
            20                  25                  30

Thr Lys Ala Asn Asn Ser Leu Ile Ala Leu Arg Phe Asn Asp Tyr Asp
        35                  40                  45

Asp Pro Val Asp Gly Asp Phe Glu Asn Leu Gly Thr Thr Val Glu Phe
    50                  55                  60

Val Pro Glu Thr Lys Asp Ala Thr Leu Thr Asn His Leu Gly Thr Tyr
65                  70                  75                  80

Asp Leu Leu Gln Phe His Phe His Trp Gly Arg Asp Ser Ser Glu Gly
                85                  90                  95

Ser Glu His Arg Ile Asp Asp Glu Gln Tyr Ser Ala Glu Ile His Phe
            100                 105                 110

Val His Leu Lys Gln Gly Ala Ser Pro Ser Asp Thr Ala Gly Asp Thr
        115                 120                 125

Phe Ser Val Val Ala Val Leu Cys Glu Ala Ala Asp Ile Pro Ile Arg

130                 135                 140

Gly Val Trp Ala Lys Leu Ser Pro Val Pro Thr Gly His Glu Asp Ser
145                 150                 155                 160

His Ser Val Ser Asp Leu Val Tyr Thr Asp Leu Leu Pro Arg Asn Arg
                165                 170                 175

Asp Tyr Tyr His Tyr Glu Gly Ser Leu Thr Thr Pro Leu Cys Asp Glu
            180                 185                 190

Thr Val Gln Trp Phe Val Leu Lys Asn Thr Ile Lys Ile Pro Lys Ala
        195                 200                 205

Phe Leu Thr Met Leu Arg Arg Val Glu Ser Asp Glu Asp Gly Thr Leu
    210                 215                 220

Leu Thr Phe Asn Phe Arg Asn
225                 230

<210>  11
<211>  36
<212>  DNA
<213>  artificial sequence

<220>
<223>  primer

<400>  11
ccgccatggg tcggtgtgat ttcaactact acaatc                                36

<210>  12
<211>  36
<212>  DNA
<213>  artificial sequence

<220>
<223>  primer

<400>  12
ccgccatggt cagccatgat ggtctctgcc gtgacg                                36

<210>  13
<211>  33
<212>  DNA
<213>  artificial sequence

<220>
<223>  primer

<400>  13
ccgccatggg cgtttgcgct tcttgccttc acc                                   33

<210>  14
<211>  5
<212>  PRT
<213>  Astrosclera willeyana

<400> 14

Asp Phe Asn Tyr Tyr
1               5